# EUROPEAN PATENT SPECIFICATION

(11) **EP 2 692 314 B1**
(45) Date of publication and mention of the grant of the patent: **09.01.2019**
(21) Application number: 12762801.4
(22) Date of filing: 07.03.2012
(51) Int. Cl.: A61F 2/82, A61F 2/95

(54) **STENT AND STENT DELIVERY SYSTEM**
STENT UND STENTEINBRINGUNGSSYSTEM
ENDOPROTHÈSE ET SYSTÈME DE POSE DE L'ENDOPROTHÈSE

(30) Priority: 29.03.2011 JP 2011072476
(43) Date of publication of application: 05.02.2014
(73) Proprietor: Terumo Kabushiki Kaisha, Tokyo 151-0072 (JP)
(72) Inventor: BABA, Takeshi, Fujinomiya-shi, Shizuoka 418-0015 (JP)
(74) Representative: Prüfer & Partner mbB Patentanwälte · Rechtsanwälte
(86) International application number: PCT/JP2012/055774
(87) International publication number: WO 2012/132801

(56) References cited:
- EP-A1- 0 887 051
- WO-A1-03/022178
- WO-A1-2007/005800
- JP-A- 2000 084 090
- JP-A- 2004 329 790
- JP-A- 2009 240 613
- JP-A- 2009 240 796
- US-A1- 2001 032 011
- US-A1- 2005 283 228
- US-A1- 2009 204 202
- US-A1- 2009 204 202

## Description

### Technical Field

The present invention relates to a stent and a stent delivery system as defined by the claims. Insofar as the terms "invention" and/or "embodiment" are used in the following, and/or features are presented as being optional, this should be interpreted in such a way that the only protection sought is that of the invention as claimed.

### Background Art

A stent delivery system is a medical device used for improvement of a stenosis or occluded part generated in a lumen in a living body. The stent delivery system is provided with an inflatable balloon which is disposed on the outer circumference of a distal end portion of a hollow shaft section, and a stent which is composed of wavy linear members, is disposed on the outer circumference of the balloon and is expanded by inflation of the balloon.

The stent is caulked (mounted) onto the balloon in a radially contracted state. After arrival in a target part (stenosis or occluded part), the balloon is inflated to bring the stent into plastic deformation, whereby the stent is put indwelling in secure contact with the inner surface of the target part. In this instance, depending on the state of the target part or the way in which the balloon is inflated, the stent may fail to spread uniformly in the circumferential direction, so that the stent may be unable to display its intended performance.

In consideration of the above-mentioned problem, for example, there is a stent wherein apex parts (opposed stop surfaces) of linear members (struts) are engaged with each other so that hinge regions of the linear members will not be opened in excess of a predetermined angle, whereby nonuniform expansion of the stent in the circumferential direction is prevented (see, for example,

Patent Document 1).

### Prior Art Document

### Patent Document

Patent Document 1: JP-T-2000-516486

### Summary of Invention

### Technical Problem

However, the material thickness of a stent is in general as extremely small as about 100 pm. In addition, a stent in practical use receives forces in various directions from a blood vessel wall or the like. In the stents like the one disclosed in Patent Document 1, therefore, it may be difficult to successfully achieve the intended engagement, and the stent may be expanded nonuniformly. Thus, there has been the problem that nonuniform expansion of the stent cannot be obviated assuredly.

WO 03/022178 discloses a stent according to the preamble of claim 1. Other stents are known from US 2009/0204202 A1 and EP 0 887 051 A1.

The present invention has been made to solve the above-mentioned problem involved in the related art. Accordingly, it is an object of the present invention to provide a stent and a stent delivery system by which uniform expansion can be achieved assuredly. This object is achieved by the stent and by the stent delivery system having the features of claims 1 and 5, respectively. The present invention is further developed as defined in the dependent claims.

### Technical Solution

In order to attain the above object, according to one aspect of the present invention, there is provided a stent disposed on an outer circumference of an inflatable balloon and expanded by inflation of the balloon, the stent including a wavy linear member which extends in the circumferential direction of the stent and can be expanded and contracted in the radial direction of the stent. The linear member includes a pair of straight parts, an apex part, and an opening. Both ends of the apex part are connected to the pair of straight parts. The opening is located at least in the apex part. Furthermore, the apex part includes a link part composed of a portion which is located to the outer circumference side relative to the opening, and a base part composed of a portion which is located to the inner circumference side relative to the opening. The base part has a curved inner circumference. The link part lies across a vertex of the base part. The width of the link part in relation to the circumferential direction of the stent is smaller than the width of the base part in relation to the circumferential direction of the stent, at the vertex of the base part.

In order to attain the above object, according to another aspect of the present invention, there is provided a stent delivery system which includes: a balloon catheter including a hollow shaft section and an inflatable balloon disposed on the outer circumference of a distal end portion of the shaft section; and the above-mentioned stent, the stent being disposed on the outer circumference of the balloon and expanded by inflation of the balloon.

### Advantageous Effect

According to the present invention, when deformation is concentrated in certain of the apex parts and nonuniform expansion is about to be generated at the time of expansion of the stent, the link part disposed at the apex part in which the deformation is concentrated displays a springy action (elasticity), whereby plastic deformation of the base part of the apex part in which the deformation is concentrated is restrained. This promotes deformation of the other apex parts (the apex parts where plastic deformation amount and link part's springy action are relatively smaller), resulting in that uniform expansion is achieved. Specifically, in the stent of the present invention, the base part has a curved inner circumference, the link part lies across the vertex of the base part, and the width of the link part in relation to the circumferential direction of the stent is smaller than the width of the base part in relation to the circumferential direction of the stent, at the vertex of the base part. This ensures that the springy action of the link part can be displayed effectively. In addition, since mechanical operational element (for example, engagement) is not utilized for uniform expansion, even if the material thickness of the stent is small or the stent receives forces in various directions from a blood vessel wall or the like in practical use of the stent, the influences of the small material thickness or the forces are limited. In short, it is possible to provide a stent and a stent delivery system by which uniform expansion can be achieved reliably.

Preferably, the outer circumference of that portion of the link part which is opposite to the vertex has a curved shape. In this case, the springy action of the link part can be displayed effectively.

Preferably, the radius of curvature of the outer circumference of the link part is greater than the radius of curvature of the inner circumference of the base part. In this case, the springy action of the link part will be favorable, and plastic deformation of the base part will proceed smoothly.

Preferably, the linear member constitutes an annular body, and a plurality of such annular bodies are arranged along the axial direction of the stent. In this case, the strength with which the stent is fixed to the balloon (stent retaining force) can be enhanced, and drop-off of the stent from the balloon can be restrained reliably.

Preferably, the annular bodies adjacent to each other along the axial direction of the stent are integrated through a connecting part. In this case, the strength with which the stent is fixed to the balloon (stent retaining force) can be enhanced, and drop-off of the stent from the balloon can be restrained assuredly.

Preferably, the linear member has the apex part connected with the connecting part and the apex part not connected with the connecting part. In this case, the apex parts will be easily deformed, so that the stent will be expanded more easily.

Further objects, features and advantages of the present invention will become apparent by referring to the preferred embodiment described below and illustrated in the accompanying drawings.

### Brief Description of Drawings

FIG. 1 is a schematic view for illustrating a stent delivery system according to an embodiment of the present invention.
FIG. 2 is a schematic view for illustrating the use for the stent delivery system shown in FIG. 1.
FIG. 3 is a sectional view for illustrating a distal end portion of the stent delivery system shown in FIG. 1.
FIG. 4 is a partial development for illustrating an initial state of a stent shown in FIG. 3.
FIG. 5 is a partial development for illustrating an expanded state of the stent shown in FIG. 3.
FIG. 6 is a conceptual drawing for illustrating the deformation of a linear member shown in FIG. 5 into an expanded state.
FIG. 7 is an illustration of modification 1 according to the embodiment of the present invention.
FIG. 8 is an illustration of modification 2 according to the embodiment of the present invention.
FIG. 9 is an illustration of modification 3 according to the embodiment of the present invention.

### Mode for Carrying Out the Invention

Now, an embodiment of the present invention will be described below referring to the drawings.

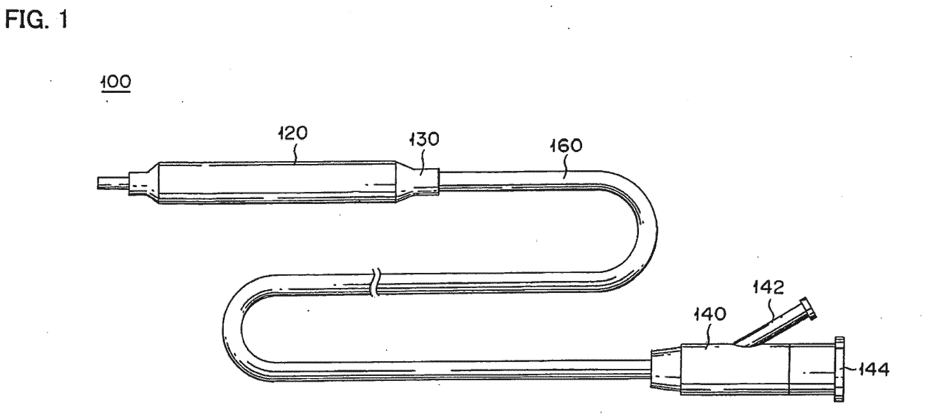

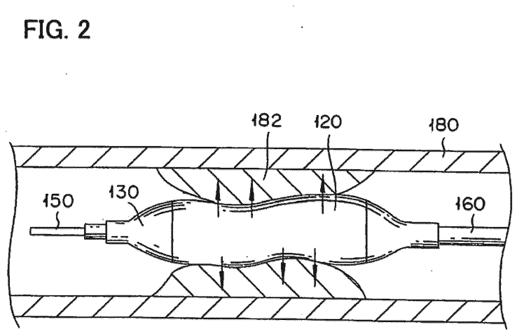

FIG. 1 is a schematic view for illustrating a stent delivery system according to an embodiment of the present invention, and FIG. 2 is a schematic view for illustrating the use for the stent delivery system shown in FIG. 1.

The stent delivery system 100 according to the embodiment of the present invention is for use of improvement of a stenosis (or occluded part) 182 generated in a lumen 180 in a living body (see FIG. 2). As shown in FIG. 1, the stent delivery system 100 includes a hollow shaft tube (shaft section) 160, a balloon 130 disposed on an outer circumference of a distal end portion of the shaft tube 160, a stent 120 disposed on an outer circumference of the balloon 130, and a hub 140 located at a proximal portion of the shaft tube 160.

The lumen 180 in the living body is, for example, a coronary artery of the heart. One of the purposes of putting the stent 120 indwelling is to prevent restenosis after PTCA (Percutaneous Transluminal coronary Angioplasty). The mode of using the stent delivery system 100 is not restricted to the mode of applying it to a stenosis generated in the coronary artery of the heart, and it may be applied also to a stenosis generated in other blood vessels, bile duct, trachea, esophagus, urethra and the like.

The stent 120 is a medical device to be put indwelling in secure contact with the inner surface of the stenosis 182, to thereby maintain the patency of the lumen 180. The stent 120 is configured to be expandable. The stent 120 is engaged with the balloon 130 so as to restrain positional deviation or peeling (separation) of the stent 120 from the balloon 130. The stent 120 is so configured that uniform expansion thereof can be securely achieved, as described later.

The balloon 130 is so configured that it can be freely inflatable so as to expand, and to enlarge the radius of, the stent 120 disposed on the outer circumference thereof.

The hub 140 has an injection port 142 and a guide wire port 144. The injection port 142 is used, for example, for introduction and discharge of a pressure fluid which is used for inflating the balloon 130. The pressure fluid is, for example, a liquid such as physiological saline and an angiographic contrast agent. The guide wire port 144 is used for inserting a guide wire 150 into the shaft tube 160 and protruding the guide wire 150 beyond a distal end portion of the shaft tube 160.

Putting the stent 120 indwelling is carried out, for example, as follows.

First, a distal end portion of the stent delivery system 100 is inserted into the lumen 180 of a patient, and is positioned into the stenosis 182, which is the target part, while the guide wire 150 having preliminarily been protruded beyond the distal end portion of the shaft tube 160 is advanced in a preceding manner. Then, the pressure fluid is introduced via the injection port 142 to inflate the balloon 130, thereby causing expansion and plastic deformation of the stent 120, whereby the stent 120 is put in secure contact with the stenosis 182 (see FIG. 2). Thereafter, the balloon 130 is deflated, to disengage the stent 120 and the balloon 130 from each other, thereby separating the stent 120 from the balloon 130. As a result, the stent 120 is set indwelling in the stenosis 182. Then, the stent delivery system 100 from which the stent 120 has thus been separated is moved backward, to be removed from the lumen 180.

Now, the distal end portion of the stent delivery system 100 will be described in detail below.

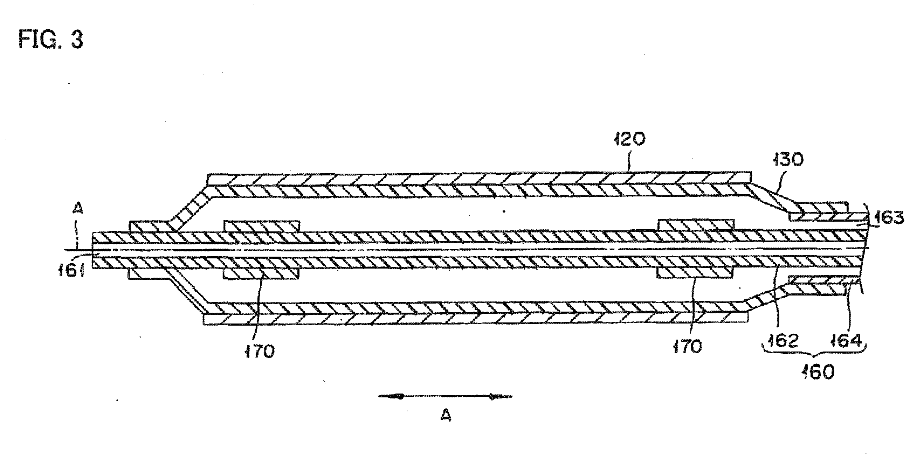

FIG. 3 is a sectional view for illustrating the distal end portion of the stent delivery system shown in FIG. 1.

The balloon 130 is disposed along the axial direction A in relation to the outer circumference of the distal end portion of the shaft tube 160, in a folded state (or a contracted state), and is freely inflatable. The stent 120 is disposed on the outer circumference of the balloon 130, in a radially contracted state. With the balloon 130 inflated, a linear member 122 of the stent 120 is expanded.

The material forming the balloon 130 is preferably a flexible material. Examples of the flexible material include polyolefin, polyvinyl chloride, polyamides, polyamide elastomers, polyurethane, polyesters such as polyethylene terephthalate, etc., polyarylene sulfides such as polyphenylene sulfide, silicone rubbers, and latex rubber. Examples of the polyolefin include polyethylene, polypropylene, ethylene-propylene copolymer, ethylene-vinyl acetate copolymer, and crosslinked ethylene-vinyl acetate copolymer.

The stent 120 is formed from a biocompatible material. Examples of the biocompatible material include nickel-titanium alloys, cobalt-chromium alloys, stainless steels, iron, titanium, aluminum, tin, and zinc-tungsten alloys.

The shaft tube 160 has an inner tube 162, and an outer tube 164 in which the inner tube 162 is inserted. The inner tube 162 communicates with the guide wire port 144 of the hub 140, and penetrates the balloon 130 to extend to the distal end of the balloon 130. Therefore, the guide wire 150 inserted in the guide wire port 144 can freely protrude beyond the distal end of the stent delivery system 100, and the inside of the inner tube 162 constitutes a guide wire lumen 161.

Cylindrical markers 170 are mounted to the outer circumference of the inner tube 162, inside the balloon 130. The markers 170 are formed from a radiopaque material, so that clear contrast images of them can be obtained under radioscopy. This ensures that the positions of the balloon 130 and the stent 120 can be easily confirmed. Examples of the radiopaque material include platinum, gold, tungsten, iridium, and their alloys.

The outer tube 164 is disposed outside the inner tube 162. A lumen 163 constituted of the space between the inner circumferential surface of the outer tube 164 and the outer circumferential surface of the inner tube 162 communicates with the injection port 142 of the hub 140. Onto the outer circumferences of distal end portions of the inner tube 162 and the outer tube 164, the balloon 130 is fixed in a liquid-tight manner. The inside of the balloon 130 communicates with the lumen 163. Therefore, the pressure fluid introduced via the injection port 142 can pass through the lumen 163, to be introduced into the inside of the balloon 130, thereby inflating the balloon 130. The method of fixing the balloon 130 to the outer circumferences of the distal end portions of the inner tube 162 and the outer tube 164 is not specifically restricted; for example, an adhesive or heat fusing is applicable.

The material constituting the outer tube 164 is preferably a flexible material. Examples of the flexible material include polyolefins such as polyethylene, polypropylene, polybutene, ethylene-propylene copolymer, ethylene-vinyl acetate copolymer, ionomers, mixtures of two or more of them, etc., thermoplastic resins such as soft polyvinyl chloride resin, polyamides, polyamide elastomers, polyesters, polyester elastomers, polyurethane, fluororesins, etc., silicone rubbers, and latex rubber.

As the material constituting the inner tube 162, the same materials as those for the outer tube 164 and metallic materials are applicable. Examples of the metallic materials include stainless steels and Ni-Ti alloys.

Incidentally, examples of the material constituting the hub 140 (see FIG. 1) include thermoplastic resins such as polycarbonate, polyamides, polysulfones, polyarylates, and methacrylate-butylene-styrene copolymer.

Now, the stent 120 will be described in detail below.

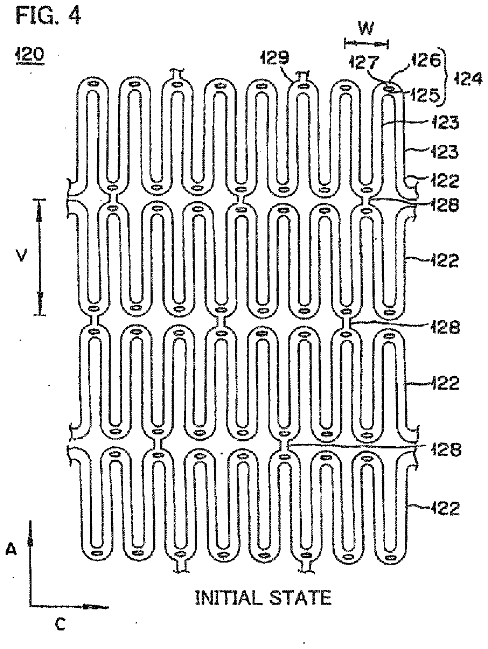

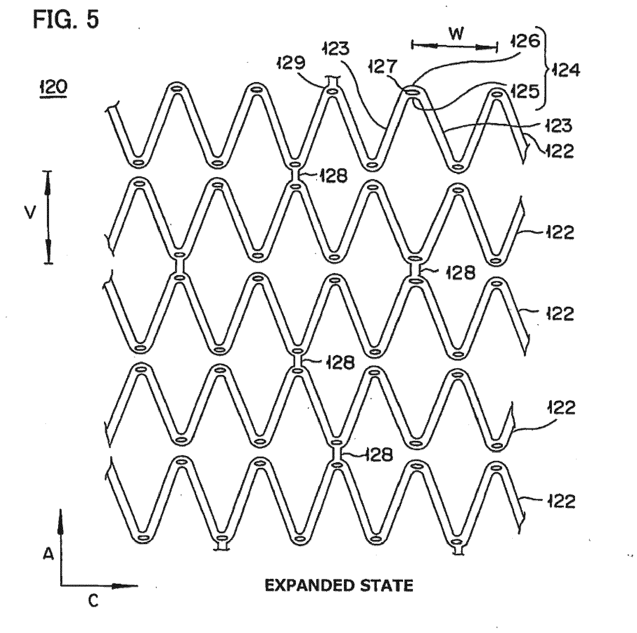

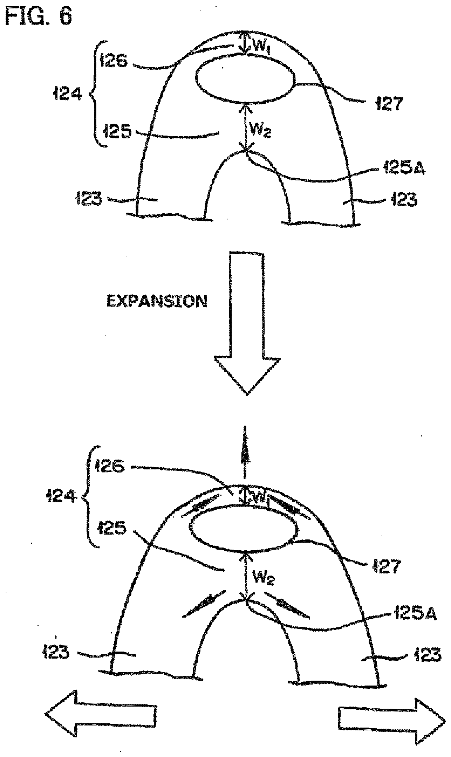

FIGS. 4 and 5 are partial developments for illustrating the stent shown in FIG. 3, in an initial state (radially contracted state) and in an expanded state, respectively. FIG. 6 is a conceptual drawing for illustrating the deformation of the linear member shown in FIG. 5 into an expanded state.

As shown in FIG. 4, the stent 120 has the linear member 122 which extends in the circumferential direction C of the stent and which can be expanded and contracted in the radial direction of the stent. The linear member 122 constitutes an annular body. A plurality of such annular bodies are arranged along the axial direction A, and the adjacent annular bodies are integrated through a connecting part 128. This ensures that the strength with which the stent is fixed to the balloon 130 (stent retaining force) is enhanced, and drop-off of the stent from the balloon 130 is securely restrained.

The linear member 122 is wavy, and includes a pair of straight parts 123, apex parts 124 and 129, and an opening 127. Both ends of each of the apex parts 124 and 129 are connected to the pair of straight parts 123. The opening 127 is located at least in each of the apex parts 124 and 129. The apex part 124 and 129 each have a link part 126 composed of a portion which is located to the outer circumference side relative to the opening 127, and a base part 125 composed of a portion which is located to the inner circumference side relative to the opening 127. Incidentally, the opening 127 is preferably provided in every one of the apex parts 124 and 129 of the linear member 122.

As shown in FIG. 4, the opening 127 is elliptic in shape. In addition, the straight part 123 is straight over the whole length thereof. Incidentally, it suffices that the straight part 123 is straight in shape at least at a part thereof, particularly in the vicinity of both ends thereof connected to the apex parts 124 and 129, respectively. Thus, the straight part 123 may have a gently curved portion at its intermediate portion, for example.

Besides, the wavelength W, or the distance between the apex points of the apex parts 124 and 129 adjacent to each other in the circumferential direction C of the stent, and the amplitude V, or the distance between the apex points of the apex parts 124 and 129 located at (connected to) both ends of the straight part 123, are substantially constant, respectively. When the stent is expanded, the linear member 122 is deformed as shown in FIG. 5. Specifically, as the radial expansion of the stent 120 progresses, end portions of the straight parts 123 are spaced away from each other, with each of the apex parts 124 and 129 as a center. This results in that the amplitude V is shortened as the wavelength W is elongated.

In this instance, when deformation is concentrated in certain of the apex parts 124 and 129 and nonuniform expansion is about to be generated, as shown in FIG. 6, the link part 126 disposed at each of the apex parts 124 and 129 in which the deformation is concentrated displays a springy action (elasticity), whereby plastic deformation of the base part 125 of each of the apex parts 124 and 129 in which the deformation is concentrated is restrained. This promotes deformation of the other apex parts (the apex parts where plastic deformation amount and link part's springy action are relatively smaller), resulting in that uniform expansion is achieved. In addition, since mechanical operational element (for example, engagement) is not utilized for uniform expansion, even if the material thickness of the stent 120 is small or the stent receives forces in various directions from a blood vessel wall or the like in practical use of the stent, the influences of the small material thickness or the forces are limited.

Therefore, uniform expansion is securely achieved. As a result, when the stent 120 is disposed in the lumen 180 so as to expand the lumen 180, for example, the force for expanding the inner surface of the lumen 180 (stenosis 182) (for example, a blood vessel expanding force) can be applied properly (see FIG. 2).

Preferably, the inner circumference of the base part 125 is made to be curved, or the link part 126 is so disposed as to lie across the vertex 125A which is an inflection point of the base part 125, or the outer circumference of the link part 126 at a portion opposite to the vertex 125A is made to be curved. In this case, the springy action of the link part 126 can be displayed effectively.

The radius of curvature of the outer circumference of the link part 126 is preferably greater than the radius of curvature of the inner circumference of the base part 125. In this case, the springy action of the link part 126 will be favorable, and plastic deformation of the base part 125 will proceed smoothly. Besides, the width W₁ of the link part 126 in relation to the circumferential direction C of the stent is preferably smaller than the width W₂ of the base part 125 in relation to the circumferential direction C of the stent, at the vertex 125A of the base part 125 (see FIG. 6). In this case, the springy action of the link part 126 will be more favorable.

As shown in FIGS. 4 and 5, the linear member 122 has the apex parts 129 which are connected with the connecting part 128 and the apex parts 124 which are not connected with the connecting part 128. Therefore, the apex parts 124 and 129 will be deformed easily, so that the stent will be expanded more easily.

The method for forming the linear member 122 is not specifically restricted. The linear member 122 can be formed, for example, by a method in which the outer circumference of a tubular stent material is subjected to laser beam cutting on the basis of the pattern of the linear member 122, or by a method in which a masking material corresponding to the pattern of the linear member 122 is disposed on the outer circumference of a tubular stent material and etching is conducted.

Now, modifications 1 to 3 will be described below.

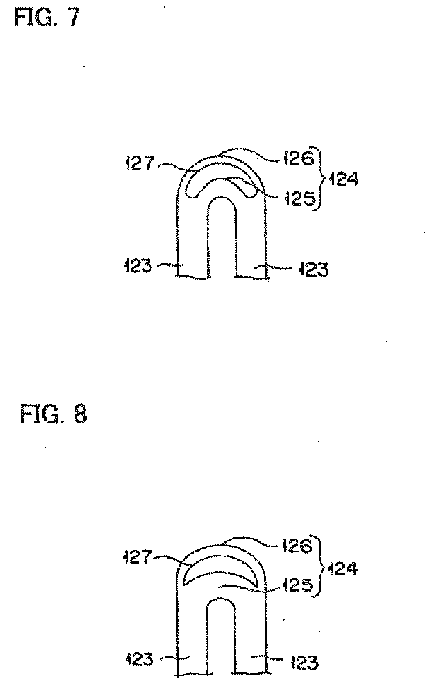

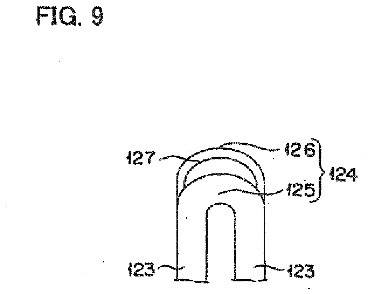

FIGS. 7 to 9 are plan views for illustrating modifications 1 to 3 according to the embodiment of the present invention.

The shape of the opening 127 is not restricted to an ellipse. For instance, the shape may be a shape obtained by curving a central portion of an ellipse (see FIG. 7) or a crescent (see FIG. 8). In addition, the link part 126 is not restricted to the one that is formed integral with the base part 125 (linear member 122); thus, a link part 126 formed as a separate body may be joined to the base part 125 (the apex part 124). Incidentally, the opening 127 may be so configured as to extend beyond the apex part 124 to the straight part 123, if necessary. In this case, the end portions of the link part 126 are connected to the straight parts 123.

As above-described, in the present embodiment, it is possible to obtain a stent and a stent delivery system by which uniform expansion can be achieved assuredly.

The present invention is not restricted to the above-described embodiment, and various alterations are possible within the scope of the claims. For instance, the balloon catheter is applicable not only to the over-the-wire (OTW) type but also to the rapid exchange (RX) type.

Besides, the stent may be formed from a biodegradable polymer which is dissolved and absorbed in a living body with the lapse of time, or the stent surface may be coated with a drug (biologically active agent). Examples of the biodegradable polymer include polylactic acid, polyglycolic acid, and lactic acid-glycolic acid copolymer. Examples of the drug include carcinostatic agents, immunosuppressors, antibiotics, immunosuppressors, and antithrombogenic agents. Furthermore, a marker may be disposed on the proximal side of the shaft tube.

### Explanation of Reference Symbols

- 100: Stent delivery system,
- 120: Stent,
- 122: Linear member,
- 123: Straight part,
- 124, 129: Apex part,
- 125: Base part,
- 125A: Vertex,
- 126: Link part,
- 127: Opening,
- 128: Connecting part,
- 130: Balloon,
- 140: Hub,
- 142: Injection port,
- 144: Guide wire port,
- 150: Guide wire,
- 160: Shaft tube (Shaft section),
- 161: Lumen,
- 162: Inner tube,
- 163: Lumen,
- 164: Outer tube,
- 170: Marker,
- 180: Lumen,
- 182: Stenosis,
- A: Axial direction,
- C: Circumferential direction,
- V: Amplitude,
- W: Wavelength,
- W₁, W₂: Width.

## Claims

1. A stent (120) disposed on an outer circumference of an inflatable balloon (130) and expanded by inflation of the balloon (130), the stent (120) comprising
a wavy linear member (122) which extends in the circumferential direction (C) of the stent (120) and can be expanded and contracted in the radial direction of the stent (120),
wherein the linear member (122) includes a pair of straight parts (123), an apex part (124, 129), and an opening (127),
both ends of the apex part (124, 129) are connected to the pair of straight parts (123),
the opening (127) is located at least in the apex part (124, 129),
the apex part (124, 129) includes a link part (126) composed of a portion which is located to an outer circumference side relative to the opening (127), and a base part (125) composed of a portion which is located to an inner circumference side relative to the opening (127),
the base part (125) has a curved inner circumference, the link part (126) lies across a vertex (125A) of the base part (125),
the width (W₁) of the link part (126) in the axial direction (A) of the stent (120) is smaller than the width (W₂) of the base part (125) in the axial direction (A) of the stent (120), at the vertex (125A) of the base part (125),
the linear member (122) constitutes an annular body, and a plurality of the annular bodies are arranged along an axial direction (A) of the stent (120), and
the stent (120) comprises connecting parts (128) each of which is disposed between the annular bodies adjacent to each other along the axial direction (A) of the stent (120), and each of which integrates the annular bodies,
**characterized in that**
the connecting parts (128) connect a link part (126) of an apex part (124, 129), which has an opening (127), of one annular body with a link part (126) of an apex part (124, 129), which has an opening (127), of another annular body.

2. The stent (120) according to claim 1, wherein a portion, opposite to the vertex (125A), of the link part (126) has a curved outer circumference.

3. The stent (120) according to claim 2, wherein the radius of curvature of an outer circumference of the link part (126) is greater than the radius of curvature of an inner circumference of the base part (125).

4. The stent (120) according to any one of claims 1 to 3, wherein the linear member (122) includes an apex part (124, 129) connected with the connecting part (128) and another apex part (124, 129) not connected with the connecting part (128) .

5. A stent delivery system (100) comprising:
a balloon catheter including a hollow shaft section (160) and an inflatable balloon (130) disposed on an outer circumference of a distal end portion of the shaft section (160); and
the stent (120) according to any one of claims 1 to 4, the stent (120) being disposed on an outer circumference of the balloon (130) and expanded by inflation of the balloon (130).

## Patentansprüche

1. Stent (120), angeordnet auf einem äußeren Umfang eines aufblasbaren Ballons (130) und durch Aufblasen des Ballons (130) expandiert, wobei der Stent (120) umfasst
ein wellenförmiges lineares Element (122), das sich in der Umfangsrichtung (C) des Stents (120) erstreckt und in der radialen Richtung des Stents (120) expandiert und zusammengezogen werden kann,
wobei das lineare Element (122) ein Paar gerader Teile (123), ein Scheitelteil (124, 129) und eine Öffnung (127) beinhaltet,
beide Enden des Scheitelteils (124, 129) mit dem Paar gerader Teile (123) verbunden sind,
die Öffnung (127) mindestens im Scheitelteil (124, 129) angeordnet ist,
das Scheitelteil (124, 129) einen Verbindungsteil (126) beinhaltet, der aus einem Abschnitt zusammengesetzt ist, der an einer Seite eines äußeren Umfangs in Bezug auf die Öffnung (127) angeordnet ist, und einem Basisteil (125), das aus einem Abschnitt zusammengesetzt ist, der an einer Seite eines inneren Umfangs in Bezug auf die Öffnung (127) angeordnet ist,
das Basisteil (125) einen gekrümmten Innenumfang aufweist,
das Verbindungsteil (126) über einem Scheitelpunkt (125A) des Basisteils (125) liegt, die Breite (W₁) des Verbindungsteils (126) in der axialen Richtung (A) des Stents (120) kleiner ist als die Breite (W₂) des Basisteils (125) in der axialen Richtung (A) des Stents (120) am Scheitelpunkt (125A) des Basisteils (125),
das lineare Element (122) einen ringförmigen Körper bildet, und eine Vielzahl der ringförmigen Körper entlang einer axialen Richtung (A) des Stents (120) angeordnet sind, und
der Stent (120) Verbindungsteile (128) umfasst, von denen jedes zwischen den ringförmigen Körpern benachbart zueinander entlang der axialen Richtung (A) des Stents (120) angeordnet ist und von denen jedes die ringförmigen Körper integriert, **dadurch gekennzeichnet, dass**
die Verbindungsteile (128) ein Verbindungsteil (126) eines Scheitelteils (124, 129), das eine Öffnung (127) aufweist, eines Ringkörpers mit einem Verbindungsteil (126) eines Scheitelteils (124, 129), das eine Öffnung (127) aufweist, eines anderen Ringkörpers verbinden.

2. Stent (120) nach Anspruch 1, wobei ein Abschnitt des Verbindungsteils (126) gegenüber dem Scheitelpunkt (125A) einen gekrümmten Außenumfang aufweist.

3. Stent (120) nach Anspruch 2, wobei der Krümmungsradius eines äußeren Umfangs des Verbindungsteils (126) größer ist als der Krümmungsradius eines inneren Umfangs des Basisteils (125).

4. Stent (120) nach einem der Ansprüche 1 bis 3, wobei das lineare Element (122) einen mit dem Verbindungsteil (128) verbundenen Scheitelabschnitt (124, 129) und einen weiteren Scheitelabschnitt (124, 129) einschließt, der nicht mit dem Verbindungsteil (128) verbunden ist.

5. Stenteinbringungssystem (100), umfassend:
einen Ballonkatheter einschließend einen Bereich eines hohlen Schaftes (160) und einen aufblasbaren Ballon (130), der auf einem äußeren Umfang eines distalen Endabschnitts des Schaftbereichs (160) angeordnet ist; und
einen Stent (120) nach irgendeinem der Ansprüche 1 bis 4, wobei der Stent (120) auf einem äußeren Umfang des Ballons (130) angeordnet und durch Aufblasen des Ballons (130) expandiert ist.

## Revendications

1. Endoprothèse (120) disposée sur une périphérie externe d'un ballonnet gonflable (130) et dilatée par gonflage du ballonnet (130), l'endoprothèse (120) comprenant
un élément linéaire ondulé (122) qui s'étend dans la direction circonférentielle (C) de l'endoprothèse (120) et peut être dilaté et contracté dans la direction radiale de l'endoprothèse (120),
dans laquelle l'élément linéaire (122) comprend une paire de parties droites (123), une partie apex (124, 129), et une ouverture (127),
les deux extrémités de la partie apex (124, 129) sont raccordées à la paire de parties droites (123),
l'ouverture (127) se trouve au moins dans la partie apex (124, 129),
la partie apex (124, 129) comprend une partie de liaison (126) composée d'une portion qui se trouve sur un côté périphérique extérieur relativement à l'ouverture (127), et une partie base (125) composée d'une portion qui se trouve sur un côté périphérique intérieur relativement à l'ouverture (127),
la partie base (125) a une périphérie interne courbée,
la partie de liaison (126) s'étend à travers un vertex (125A) de la partie base (125),
la largeur (W₁) de la partie de liaison (126) dans la direction axiale (A) de l'endoprothèse (120) est plus courte que la largeur (W₂) de la partie base (125) dans la direction axiale (A) de l'endoprothèse (120), au niveau du vertex (125A) de la partie base (125),
l'élément linéaire (122) constitue un corps annulaire, et une pluralité des corps annulaires sont disposés le long d'une direction axiale (A) de l'endoprothèse (120), et
l'endoprothèse (120) comprend des parties de raccordement (128), chacune d'entre elles est disposée entre les corps annulaires adjacents les uns aux autres dans la direction axiale (A) de l'endoprothèse (120), et chacune d'entre elles intègre les corps annulaires,
**caractérisé en ce que**
les parties de raccordement (128) se raccordent à une partie de liaison (126) d'une partie apex (124, 129), qui a une ouverture (127), d'un corps annulaire qui est une partie de liaison (126) d'une partie apex (124, 129), qui a une ouverture (127), d'un autre corps annulaire.

2. Endoprothèse (120) selon la revendication 1, dans laquelle une portion, opposée au vertex (125A), de la partie de liaison (126) a une circonférence externe incurvée.

3. Endoprothèse (120) selon la revendication 2, dans laquelle le rayon de courbure d'une périphérie externe de la partie de liaison (126) est plus grand que le rayon de courbure d'une périphérie interne de la partie base (125).

4. Endoprothèse (120) selon l'une quelconque des revendications 1 à 3, dans laquelle l'élément linéaire (122) comprend une partie apex (124, 129) raccordée à la partie de raccordement (128) et une autre partie apex (124, 129) non raccordée à la partie raccordement (128).

5. Système de pose d'endoprothèse (100) comprenant :
un cathéter à ballonnet comprenant une section tige (160) creuse et un ballonnet gonflable (130) disposé sur une périphérie externe d'une partie extrémité distale de la section tige (160) ; et
l'endoprothèse (120) selon l'une quelconque des revendications 1 à 4, l'endoprothèse (120) étant disposée sur une périphérie externe du ballonnet (130) et dilatée par gonflage du ballonnet (130).
